# EUROPÄISCHE PATENTSCHRIFT

(11) **EP 2 194 872 B1**
(45) Veröffentlichungstag und Bekanntmachung des Hinweises auf die Patenterteilung: **02.11.2011**
(21) Anmeldenummer: 08801620.9
(22) Anmeldetag: 19.08.2008
(51) Int. Cl.: A61B 5/151

(54) **STECHSYSTEM**
PIERCING SYSTEM
SYSTÈME PIQUEUR

(30) Priorität: 28.08.2007 EP 07016808
(43) Veröffentlichungstag der Anmeldung: 16.06.2010
(73) Patentinhaber: Roche Diagnostics GmbH, 68305 Mannheim (DE); F. Hoffmann-La Roche AG, 4070 Basel (CH)
(72) Erfinder: KONYA, Ahmet, 67165 Waldsee (DE); SCHÖTTLE, Klaus, 77731 Willstätt (DE)
(74) Vertreter: Twelmeier Mommer & Partner
(86) Internationale Anmeldenummer: PCT/EP2008/006797
(87) Internationale Veröffentlichungsnummer: WO 2009/030359

(56) Entgegenhaltungen:
- EP-A- 1 790 288
- US-A1- 2005 245 845

## Beschreibung

Die Erfindung betrifft ein Stechsystem mit einem Lanzettenträgerband, das mehrere Lanzetten trägt, einer Transporteinrichtung, um das Lanzettenträgerband in einer Förderrichtung entlang eines Förderwegs zu transportieren und dadurch Lanzetten des Lanzettenträgerbandes nacheinander in eine Stechposition zu bringen, und einem Stechantrieb, um eine Lanzette, die sich in der Stechposition befindet, in eine Stechbewegung zu versetzen. Ein derartiges Stechsystem ist beispielsweise aus der DE 28 03 345 B1 bekannt.

Bei dem bekannten Stechsystem wird das Lanzettenträgerband mittels Stiften, die durch Löcher in dem Band hindurch greifen, für einen Stich arretiert und die Lanzette dann relativ zu dem Band in dessen Querrichtung verschoben.

Nachteilig an dem bekannten Stechsystem ist dessen aufwendige Mechanik, die recht große Abmessungen der Lanzetten und anderen Systemkomponenten erforderlich macht.

Ein Stechsystem gemäß Oberbegriff des Anspruchs 1 ist aus EP-1790288 bekannt

Aufgabe der Erfindung ist es deshalb, einen Weg aufzuzeigen, wie ein kompaktes und kostengünstiges Stechsystem mit einem Lanzettenträgerband geschaffen werden kann.

Diese Aufgabe wird durch ein Stechsystem mit den im Anspruch 1 angegebenen Merkmalen gelöst. Vorteilhafte Weiterbildungen der Erfindung sind Gegenstand der Unteransprüche.

Bei einem erfindungsgemäßen Stechsystem wird eine in eine Stechposition gebrachte Lanzette zusammen mit einem diese Lanzette tragenden Abschnitt des Lanzettenträgerbandes in Stichrichtung bewegt. Aufwendige Maßnahmen zum Arretieren des Lanzettenträgerbandes und einem Verschieben der Lanzette relativ zu dem Band können deshalb entfallen. Die erfindungsgemäße Maßnahme, bei einem Stich die Lanzette zusammen mit dem Lanzettenträgerband in Stichrichtung zu bewegen, führt jedoch zu dem Problem, dass das Lanzettenträgerband relativ zu einem Gerätegehäuse in Stichrichtung beweglich sein muss. Dabei darf das Lanzettenträgerband nicht reißen. Insbesondere muss das Lanzettenträgerband für einen möglichst schmerzarmen Stich zusammen mit der stechenden Lanzette sehr schnell bewegt werden.

Erfindungsgemäß wird dieses Problem dadurch gelöst, dass mindestens ein in Förderrichtung hinter der Stechposition angeordnetes Teil der Transporteinrichtung, nachdem eine Lanzette in die Stechposition gebracht wurde, vor oder während einer Stechbewegung dieser Lanzette eine Bewegung ausführt.

Beispielsweise kann die Transporteinrichtung eine Wickeleinrichtung umfassen, wobei die Wickeleinrichtung zunächst ein Vorwärtsdrehschritt ausführt, um eine Lanzette in die Stechposition zu bringen, und danach einen Rückwärtsdrehschritt ausführt, um für eine Stechbewegung das in die Stechposition gebrachte Lanzetteband von der Wickeleinrichtung abzuwickeln.

Bei dem Vorwärtsdrehschritt wird eine Lanzette in die Stechposition gezogen, das Band also gestrafft. Durch einen anschließenden Rückwärtsdrehschritt wird das Band von der Wickeleinrichtung abgewickelt und so eine ausreichende Bandlänge für die Stechbewegung zur Verfügung gestellt. Dabei ist es möglich, den Rückwärtsdrehschritt vor dem Auslösen der Stechbewegung durchzuführen, so dass das Lanzettenträgerband zwischen der Stechposition und der Wickeleinrichtung locker und entspannt ist. Der die Lanzette in der Stechposition tragende Bandabschnitt kann dann bei der Stechbewegung retativ zu der Wickeleinrichtung problemlos bewegt werden. Der Rückwärtsdrehschritt kann in einem solchen Fall von einem rückwärts bewegten Antrieb der Wickeleinrichtung durchgeführt werden.

Möglich ist es auch, dass der Rückwärtsdrehschritt während der Stechbewegung erfolgt. Beispielsweise kann ein Antriebselement, das bei dem Vorwärtsdrehschritt zum Antreiben der Wickeleinrichtung mit der Wickeleinrichtung koppelt, für den Rückwärtsdrehschritt von der Wickeleinrichtung entkoppelt werden. Die Wickeleinrichtung kann sich dann frei drehen und einem während der Stechbewegung auftretenden Zug durch Rückwärtsdrehen nachgeben, so dass die für eine Stechbewegung benötigte Bandlänge freigegeben wird. Das Antriebselement kann beispielsweise eine Schaltkupplung, beispielsweise eine selbstschaltende Kupplung wie ein Freilauf sein, die ein Drehmoment für eine Vorwärtsdrehung der Wickeleinrichtung überträgt, jedoch bei einer Rückwärtsdrehung entkoppelt, so dass sich die Wickeleinrichtung frei rückwärts drehen kann. Möglich ist es auch eine fremdgeschaltete Kupplung zu verwenden, beispielsweise eine Klauenkupplung, die durch Betätigen des Stechantriebs geöffnet wird.

Die erfindungsgemäße Bewegung des mindestens einen Teils der Transporteinrichtung kann beispielsweise auch von einem Bandführungselement ausgeführt werden, das in einer Biegung des Förderwegs angeordnet ist und um welches das Lanzettenträgerband gebogen ist. Ein solches Bandführungselement kann als Teil der Transporteinrichtung durch eine Bewegung die Biegung des Bandes reduzieren und so Bandlänge für eine Stichbewegung freigeben. Bevorzugt befindet sich die Stechposition zwischen zwei beweglichen Bandführungselementen, um die das Lanzettenträgerband gebogen ist.

Vorzugsweise ist das mindestens eine Bandführungselement zwischen einer ersten Position, in der das Lanzettenträgerband mit einem ersten Biegewinkel um das Bandführungselement gebogen ist, und einer zweiten Position, in der das Lanzettenträgerband weniger stark oder gar nicht gebogen ist, beweglich und wird nach dem Positionieren einer Lanzette in der Stechposition hin zur zweiten Position bewegt wird.

Die erfindungsgemäße Beweglichkeit des Bandführungselements kann beispielsweise dadurch erreicht werden, dass es verschiebbar gelagert ist. Auf diese Weise kann das Bandführungselement einer durch eine Stechbewegung erhöhten Bandspannung durch eine Verschiebebewegung gegen eine Federkraft nachgeben.

Die erfindungsgemäße Bewegung des mindestens einen Teils der Transporteinrichtung kann auch eine Schwenkbewegung sein, die beispielsweise bei einer Stechbewegung von zwei Schwenkarmen ausgeführt wird, zwischen denen sich die Stechposition befindet und die jeweils ein Bandführungselement aufweisen, das in einer Biegung des Förderwegs angeordnet ist und um welches das Lanzettenträgerband gebogen ist.

Beispielsweise können die beiden Schwenkarme mit ihren Bandführungselementen eine U-förmige Bandführung bewirken, die bei einem Stich um eine geometrische Achse geschwenkt wird, die senkrecht zur Stechrichtung orientiert ist. Der die stechende Lanzette tragende Bandabschnitt kann die Stechbewegung ebenfalls als Schwenkbewegung oder als lineare Bewegung durchführen.

Die Lanzetten sind auf dem Lanzettenträgerband quer zu dessen Längsrichtung orientiert. Zwischen den Lanzetten können auf dem Lanzettenträgerband Testfelder zur Untersuchung einer durch einen Lanzettenstich gewonnen Körperflüssigkeitsprobe angeordnet sein. Zur Aufnahme einer Körperflüssigkeitsprobe kann dann ein Testfeld in die Stechposition gebracht werden und der Stechantrieb zum Ausführen einer Stechbewegung genutzt werden, durch welche das Testfeld zu einer zuvor mit einem Lanzettenstich erzeugten Stichwunde bewegt wird.

Bevorzugt wird das Lanzettenträgerband von einem Stechantrieb bei einem Lanzettenstich derart bewegt, dass das Lanzettenträgerband mit der stechenden Lanzette verbunden bleibt. Vorzugsweise wird das Lanzettenträgerband bei einer Stechbewegung mit einer auf seine in Stichrichtung vordere Längskante einwirkenden Biegeeineinrichtung gebogen, so dass sich die Spitze der Lanzette von dem Lanzettenträgerband abhebt. Mit einem von der Spitze abgewandten, in Stichrichtung hinteren Teil verbleibt die Lanzette dabei während der gesamten Stichbewegung an dem Lanzettenträgerband.

Die Transporteinrichtung umfasst vorzugsweise eine Wickeleinrichtung die durch schrittweises Aufwickeln des Lanzettenträgerbandes Lanzetten nacheinander in die Stechposition bringt. Die Stechbewegung kann parallel zur geometrischen Achse, um welche die Wickeleinrichtung drehbar ist, oder senkrecht dazu erfolgen.

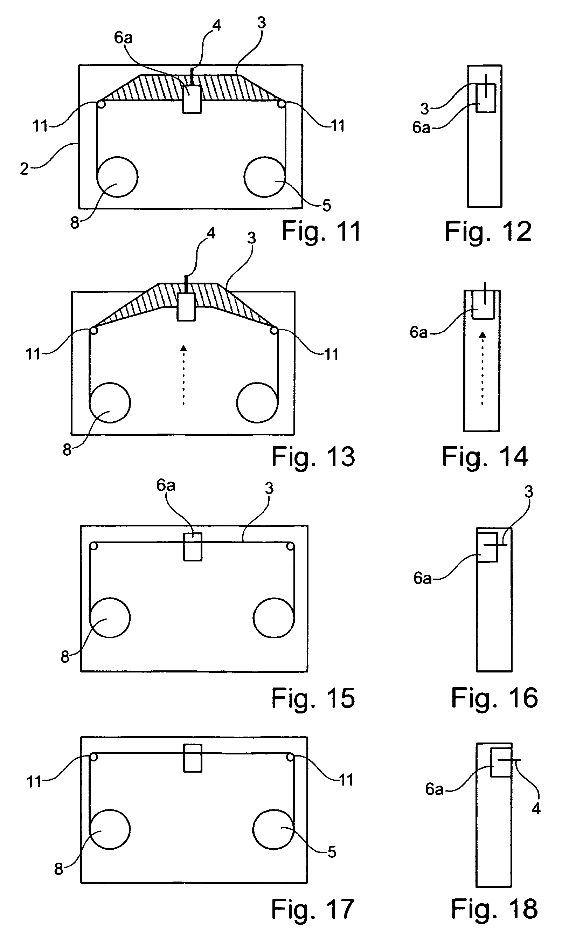

Weitere Einzelheiten und Vorteile der Erfindung werden anhand von Ausführungsbeispielen unter Bezugnahme auf die beigefügten Zeichnungen erläutert. Die dabei erläuterten Merkmale können Einzeln oder in Kombination zum Gegenstand von Ansprüchen gemacht werden. Gleiche und einander entsprechende Bauteile sind in den beigefügten Zeichnungen mit übereinstimmenden Bezugszahlen gekennzeichnet. Es zeigen: wobei die Bespiele gemäß Figuren 11-18 nicht Teil der Erfindung sind
- Figur 1:: eine schematische Darstellung eines Ausführungsbeispiels vor einer Stechbewegung;
- Figur 2:: eine Seitenansicht zu Figur 1;
- Figur 3:: das in Figur 1 dargestellte Ausführungsbeispiel beim Ausführen einer Stechbewegung;
- Figur 4:: eine Seitenansicht zu Figur 3;
- Figur 5:: eine schematische Darstellung eines weiteren Ausführungsbeispiels eines Stechsystems vor einer Stechbewegung;
- Figur 6:: eine Seitenansicht zu Figur 5;
- Figur 7:: das in Figur 5 gezeigte Ausführungsbeispiel beim Ausführen einer Stechbewegung;
- Figur 8:: eine Seitenansicht zu Figur 7;
- Figur 9:: eine schematische Darstellung eines weiteren Ausführungsbeispiels eines Stechsystems vor einer Stechbewegung;
- Figur 10:: das in Figur 9 gezeigte Ausführungsbeispiel beim Ausführen einer Stechbewegung;
- Figur 11:: eine schematische Darstellung eines Ausführungsbeispiels eines Stechsystems vor einer Stechbewegung;
- Figur 12:: eine Seitenansicht zu Figur 11;
- Figur 13:: das in Figur 11 gezeigte Ausführungsbeispiel beim Ausführen einer Stechbewegung;
- Figur 14:: eine Seitenansicht zu Figur 13;
- Figur 15:: eine schematische Darstellung eines Ausführungsbeispiels eines Stechsystems vor dem Ausführen einer Stechbewegung;
- Figur 16:: eine Seitenansicht zu Figur 15;
- Figur 17:: das in Figur 15 gezeigte Ausführungsbeispiel beim Ausführen einer Stechbewegung; und
- Figur 18:: eine Seitenansicht zu Figur 17.

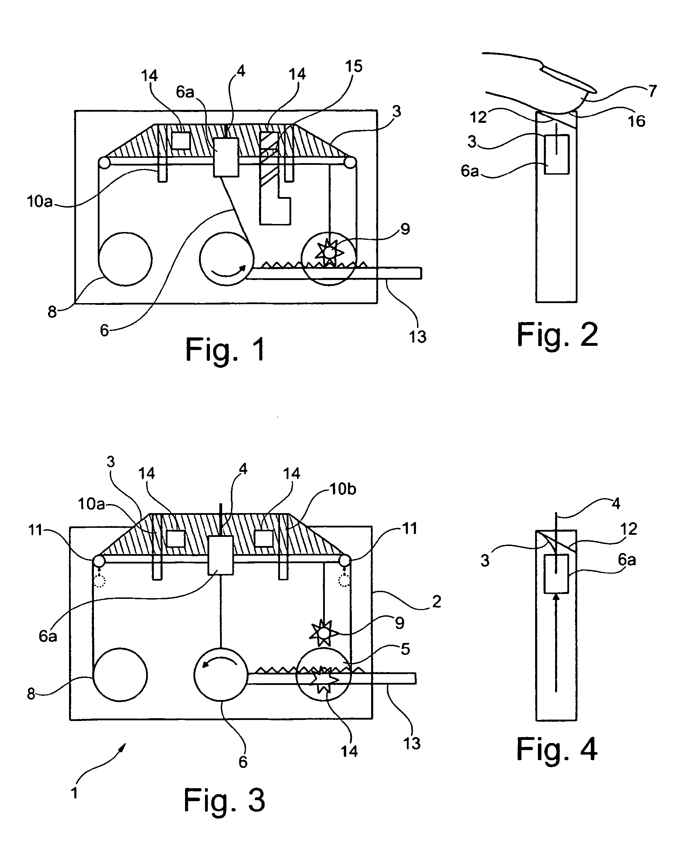

Figur 1 zeigt ein Ausführungsbeispiel eines Stechsystems 1 in einer schematischen Darstellung bei geöffnetem Gehäuse 2. Zu dem Stechsystem 1 gehört ein Lanzettenträgerband 3, das mehrere Lanzetten 4 trägt, die quer zur Längsrichtung des Bandes 3 angeordnet sind. Mit einer Wickeleinrichtung 5, die bei dem dargestellten Ausführungsbeispiel als eine angetriebene Rolle ausgebildet ist, kann das Lanzettenträgerband 3 aufgewickelt und dabei in seiner Längsrichtung bewegt werden, so dass die Lanzetten 4 des Lanzettenträgerbandes 3 nacheinander in eine Stechposition gelangen, in der sie zusammen mit dem sie tragenden Bandabschnitt mittels eines Stechantriebs 6 für eine Stechbewegung beschleunigt werden können, um in ein an eine Gehäuseöffnung 16 angelegtes Körperteil 7 einzustechen, das in Figur 2, einer Seitenansicht zu Figur 1, dargestellt ist.

Das Lanzettenträgerband 3 ist bei dem dargestellten Ausführungsbeispiel ähnlich wie bei einer Tonbandkassette auf eine Vorratsrolle 8 aufgewickelt, von der es abgewickelt und schrittweise auf die angetriebene Rolle der Wickeleinrichtung 5 aufgewickelt wird. Das Lanzettenträgerband 3 kann austauschbar in einem Gehäuse 2 eines Stechgeräts angeordnet sein, beispielsweise als Teil einer Kassette, oder in einem Gerät fest eingebaut sein, das bestimmungsgemäß entsorgt wird, sobald alle Lanzetten 4 des Lanzettenträgerbands 3 aufgebraucht sind. Anstelle der Vorratsrolle 8 kann beispielsweise auch ein Stapel verwendet werden, zu dem das Lanzettenträgerband 3 zickzack-förmig gefaltet ist.

Beim Bandtransport läuft das Lanzettenträgerband 3 mit einer Lanzette 4, bevor diese die Stechposition erreicht, an einer Umlenkeinrichtung 10a vorbei, die ein Verdrehen des Lanzettenträgerbandes 3 um 90° bewirkt. Nach Gebrauch einer Lanzette 4 wird der betreffende Abschnitt des Trägerbandes 3 an einer zweiten Umlenkeinrichtung 10b vorbeigeführt, mit der das Trägerband wieder in seine ursprüngliche Orientierung zurückgedreht wird. Das Lanzettenträgerband 3 wird auf diesem Weg mittels Führungselementen 11 geführt, zwischen denen sich die Stechposition befindet. Die Führungselemente 11 sind bevorzugt als Stifte ausgeführt. Möglich ist es auch Rollen zu verwenden, um einen möglichst reibungsarmen Bandtransport zu ermöglichen.

Die Wickeleinrichtung 5, die Führungselemente 11 und die Umlenkeinrichtungen 10a, 10b bilden bei diesem Ausführungsbeispiel die Transporteinrichtung, mit der das Lanzettenträgerband 3 in einer Förderrichtung entlang eines Förderwegs transportiert wird, um Lanzetten 4 des Lanzettenträgerbandes 3 nacheinander in die Stechposition zu bringen

Figur 2 zeigt eine schematische Seitenansicht zu Figur 1 mit einem an die Gehäuseöffnung 16 angelegten Körperteil 7, nämlich einem Finger, in dem zur Gewinnung einer Körperflüssigkeitsprobe eine Stichwunde erzeugt werden soll.

Bei einem Vorwärtsdrehschritt der Wickeleinrichtung 5 wird das Lanzettenträgeband 3 in Längsrichtung bewegt, bis eine Lanzette 4 in die in Figur 1 gezeigte Stechposition gelangt. Beispielsweise kann die Lanzette 4 eine Erhebung auf dem Trägerband 3 bilden, die an einen Anschlag des Kopplungskopfes 6a des Stechantriebs 6 anschlägt, wenn die Lanzette 4 die Stechposition erreicht hat. Beispielsweise kann der Kopplungskopf 6a aus zwei zueinander beweglichen Klemmflächen gebildet sein, zwischen denen ein in einer geöffneten Stellung ein Spalt ist, durch das Lanzettenträgerband 3 geführt ist, wobei eine Lanzette 4 in der Stechposition an einer Engstelle des Spalts anstößt. Bei einem Einstich werden die beiden Klemmflächen des Kopplungskopfes zusammengepresst, um die Lanzette 4 und das Lanzettenträgerband 3 sicher zu fassen. Nach einem Stich werden die Klemmflächen voneinander wegbewegt, so dass sich der Spalt so weit öffnet, dass die nun benutzte Lanzette 4 den Spalt passieren kann.

Die Wickeleinrichtung 5 wird über ein Antriebselement 9 angetrieben, das beim Vorwärtsdrehen der Wickelrolle, wie es zum Positionieren einer Lanzette 4 in der Stechposition erfolgt, mit der Wickeleinrichtung 5 koppelt. Bei dem dargestellten Ausführungsbeispiel ist das Antriebselement 9 ein Zahnrad, das ein Drehmoment zum Vorwärtsdrehen der Wickeleinrichtung 5 überträgt, jedoch bei einer Stechbewegung von der Wickeleinrichtung 5 entkoppelt, so dass sich diese frei rückwärts drehen kann.

Das Antriebselement 9 ist in Figur 1 in seiner an die Wickeleinrichtung 5 gekoppelten Position und in Figur 3 in seiner entkoppelten Position gezeigt. Das Antriebselement 9 ist an den Antriebskopf 6a gekoppelt, bei dem gezeigten Ausführungsbeispiel über eine Stange, und wird bei einer Stechbewegung in Stichrichtung bewegt. In seiner Kopplungsposition liegt das als Zahnrad, genauer gesagt als Zahnwalze, ausgebildete Antriebselement 9 auf einer Zahnstange 13 und auf einem in Figur 3 dargestellten Antriebszahnrad 14 der Wickeleinrichtung 5 auf. Das Antriebszahnrad 14 der Wickeleinrichtung 5 liegt neben der Zahnstange 13, so dass das Antriebselement sowohl mit der Zahnstange 13 als auch mit dem Antriebszahnrad 14 koppelt.

Zum Betätigen der Wickeleinrichtung für die Positionierung einer Lanzette 4 in der Stechposition wird die aus dem Gehäuse 2 herausragende Zahnstange 13 von einem Benutzer nach innen geschoben. Die Bewegung der Zahnstange 13 überträgt sich über das Antriebselement 9 und das nicht gezeigte Antriebszahnrad der Wickeleinrichtung 5 auf die Wickeleinrichtung, so dass sich diese dreht. Bei einem Stich wird das Antriebselement 9 zusammen mit dem Kopplungskopf 6a in Stichrichtung bewegt und auf diese von der Zahnstange und dem Antriebszahnrad der Wickeleinrichtung abgehoben, so dass es von der Wickeleinrichtung 5 entkoppelt ist und sich diese frei drehen kann.

Bei einer Stechbewegung ist das Antriebselement, das zum Vorwärtsdrehen mit der Wickeleinrichtung 5 gekoppelt ist, von der Wickeleinrichtung 5 entkoppelt, so dass die Wickeleinrichtung 5 sich rückwärts frei drehen kann. Bei einer Stechbewegung kann sich das Lanzettenträgerband 3 deshalb sowohl von der Vorratsrolle 8 als auch von der Wickeleinrichtung 5 abwickeln und erhält auf diese Weise eine ausreichende Beweglichkeit für einen bei der Stechbewegung ausgeführten Hub.

Die Zahnstange 13 dient zusätzlich auch zum Spannen einer nicht dargestellten Antriebsfeder des Stechantriebs 6. Hierfür greift ein nicht dargestelltes Antriebselement des Stechantriebs 6 in die Zähne der Zahnstange 13 ein.

Die Wickeleinrichtung 5 koppelt über eine Rutschkupplung mit dem Antriebselement 9. Auf diese Weise kann die Zahnstange stets denselben Weg in das Gerätegehäuse 2 hinein geschoben werden. Sobald eine Lanzette 4 die Stechposition erreicht hat, blockiert das Band 3 und die Rutschkupplung entkoppelt die Wickeleinrichtung 5 von der weiteren Bewegung der Zahnstange 13.

Figur 3 zeigt das in Figur 1 dargestellte Ausführungsbeispiel bei einer Stichbewegung, nämlich im Umkehrpunkt der Bewegung bei maximalem Hub des Stechantriebs, und Figur 4 eine dazugehörende Seitenansicht.

Bei dem dargestellten Ausführungsbeispiel werden bei einer Stechbewegung auch die beiden Führungselemente 11 in Stichrichtung mitbewegt. Diese Maßnahme hat den Vorteil, die Beweglichkeit des Bandes 3 zu erhöhen, so dass eine möglichst schnelle und damit schmerzarme Stechbewegung zur Gewinnung einer Körperflüssigkeitsprobe durchgeführt werden kann.

Bei einer Stechbewegung wird die in Stichrichtung vordere Längskante des Bandes 3 umgebogen, so dass sich die Spitze der stechenden Lanzette 4 von dem Band 3 abhebt und ohne durch das Band 3 behindert zu sein in ein an die Geräteöffnung 9 angelegtes Körperteil 7 eindringen kann. Das Biegen der Längskante des Bandes 3 kann beispielsweise durch eine Schrägfläche 12, beispielsweise ein Steg, bewirkt werden, die seitlich von der Stichposition angeordnet ist, so dass die Bandkante bei einer Stechbewegung auf die Schrägfläche trifft und abgelenkt wird. Eine geeignete Biegeeinrichtung mit einer Schrägfläche 12 ist in den Figuren 2 und 4 dargestellt. In den Figuren 1 und 3 ist die Biegeeinrichtung zur Vereinfachung nicht eingezeichnet.

Das Lanzettenträgerband 3 trägt zwischen den Lanzetten 4 Testfelder 14 zur Untersuchung einer durch einen Lanzettenstich gewonnenen Körperflüssigkeitsprobe. Nach einem Stich führt die Wickeleinrichtung 5 einen weiteren Vorwärtsdrehschritt aus, um ein Testfeld 14 in die Stechposition zu bringen. Anschließend führt der Kopplungskopf 6a des Stechantriebs 6 eine weitere Stechbewegung aus, bei der das Testfeld 14 zur Aufnahme einer Körperflüssigkeitsprobe zu der zuvor mit einem Lanzettenstich erzeugten Stichwunde eines an die Gehäuseöffnung 9 angelegten Körperteils 7 bewegt wird. Bei dieser Stechbewegung führt die Wickeleinrichtung 5 ebenso wie bei einer mit einer Lanzette 4 ausgeführten Stechbewegung einen Rückwärtsdrehschritt aus, um die erforderliche Bandbeweglichkeit zu bewirken.

Nach einer Probenaufnahme durch das Testfeld 14 führt die Wickeleinrichtung 5 einen weiteren Vorwärtsdrehschritt aus, um das Testfeld 14 mit der aufgenommenen Körperflüssigkeitsprobe in eine Untersuchungsposition zu bewegen, in der die Körperflüssigkeitsprobe mit einer Messeinrichtung 15 des Stechsystems untersucht wird. Beispielsweise können die Testfelder 14 Nachweisreagenzien enthalten, die mit einem in der Körperflüssigkeitsprobe enthaltenen Analyten reagieren und dabei eine Farbänderung bewirken. Diese Farbänderung kann photometrisch zur Bestimmung der Analytkonzentration, beispielsweise der Glukosekonzentration, ausgewertet werden. Die Messeinrichtung 15 kann als photometrische Messeinrichtung ausgebildet sein, in die eine Auswerteeinheit zur Konzentrationsbestimmung integriert ist. Untersuchungsergebnisse können mit einer Anzeigeeinrichtung, beispielsweise einer Flüssigkristallanzeige, die an der Außenseite des Gerätegehäuses 2 angeordnet ist, angezeigt werden.

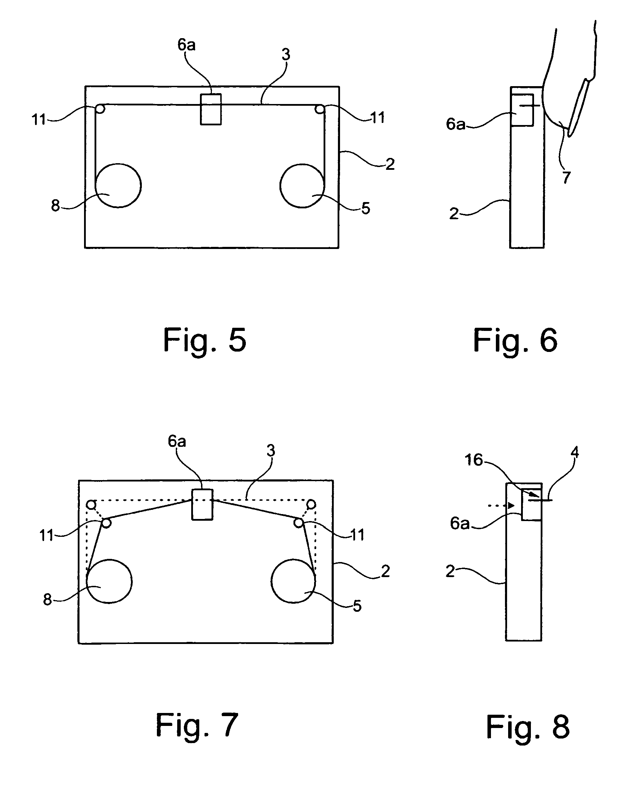

In den Figuren 5 bis 8 ist schematisch ein weiteres Ausführungsbeispiel eines Stechsystems dargestellt, das sich von dem vorhergehend beschriebenen Ausführungsbeispiel dadurch unterscheidet, dass die Stichrichtung parallel zu einer geometrischen Drehachse erfolgt, um welche sich die Wickeleinrichtung 5 dreht. Bei diesem Ausführungsbeispiel wird das Körperteil 7, in dem eine Stichwunde erzeugt werden soll, an eine an der Oberseite des Gehäuses 2 angeordnete Gehäuseöffnung 16 angelegt, während sich die entsprechende Geräteöffnung 9 bei dem Ausführungsbeispiel der Figuren 1 bis 4 an einer Schmalseite befindet. Eine Umlenkeinrichtung zum Verdrehen des Lanzettenträgerbandes 3 ist bei dem in den Figuren 5 bis 8 dargestellten Ausführungsbeispiel folglich nicht vorhanden, so dass sich die Bandführung entsprechend vereinfacht.

Im Übrigen könnte dieses Ausführungsbeispiel ebenso wie das vorstehend beschriebene Ausführungsbeispiel ausgebildet sein, insbesondere die Wickeleinrichtung 5 durch einen Rückwärtsdrehschritt für Bandbeweglichkeit bei einer Stechbewegung sorgen.

Bei dem in den Figuren 5 bis 8 schematisch dargestellten Ausführungsbeispiel wird die Bandbeweglichkeit für eine Stechbewegung jedoch durch eine Bewegung der Führungselemente 11 bewirkt. In Figur 5 sind die Führungselemente 11 in einer ersten Position dargestellt, in der das Lanzettenvorratsband 3 mit einem Biegewinkel von 70° bis 110°, vorzugsweise 90°, um die Führungselemente 11 gebogen ist. In dieser ersten Position befinden sich die Führungselemente 11, wenn dass Lanzettenvorratsband 3 in Förderrichtung bewegt wird, um eine neue Lanzette 4 in die Stechposition zu bringen.

In Figur 7 sind die Führungselemente 11 in einer zweiten Position dargestellt, in der das Lanzettenvorratsband 3 mit einem kleineren Biegewinkel um die Führungselemente 11 gebogen ist. Zur Verdeutlichung des Unterschieds sind in Figur 7 zusätzlich auch die Führungselemente 11 in der zweiten Position dargestellt. Bei einem Stich befinden sich die Führungselemente 11 in der zweiten Position.

Die Führungselemente 11 sind verschiebbar gelagert, so dass sie zwischen der ersten und der zweiten Position beweglich sind. Möglich ist es, die Führungselemente 11 mit einer Feder zu koppeln, die bei einer Verschiebung aus der ersten Position in Richtung zur zweiten Position eine Rückstellkraft bewirkt. Eine Erhöhung der Bandspannung, insbesondere als Folge einer Stechbewegung, bewirkt, dass sich die Führungselemente 11 hin zur zweiten Position verschieben. Die erste und die zweite Position können dabei Extremalpositionen bilden, die nur selten oder nie eingenommen werden, da auch bei einem Bandtransport eine geringe Spannung existieren kann, die bereits eine geringfügige Auslenkung aus der ersten Position bewirkt.

Möglich ist es auch, die Führungselemente 11 aktiv zwischen der ersten und der zweiten Position zu bewegen, um für eine Stechbewegung Bandbeweglichkeit zu erreichen. Beispielsweise können die Führungselemente 11 mittels eines Antriebs aus der ersten in die zweite Position bewegt werden, nachdem eine Lanzette 4 in die Stechposition gelangt ist, aber bevor ein Stich ausgelöst wird.

Beweglich Bandführungselemente 11, wie sie vorstehend beschrieben sind, können anstelle einer rückdrehbaren Wickeleinrichtung auch bei einer Bandführung gemäß dem Ausführungsbeispiel der Figuren 1 bis 4 verwendet werden.

Ein Vorteil beweglicher Bandführungselemente 11 ist, dass die Wickeleinrichtung 5 mit einer Rückdrehsperre versehen werden kann. Auch ist es leichter möglich, eine Zahnstange 13, wie sie in Figuren 1 und 3 gezeigt ist, über eine Rutschkupplung an die Wickeleinrichtung 5 zu koppeln.

Bei dem in den Figuren 5 bis 8 gezeigten Ausführungsbeispiel kann zur Minimierung von Reibungsmomenten ergänzend vorgesehen sein, dass die Führungselemente 11 auch in Stichrichtung beweglich sind. Hierfür ist es günstig, die Führungselemente 11 als Hülsen auszuführen, die in Stichrichtung verschieblich gelagert sind, beispielsweise auf Lagerzapfen, um welche sich die Hülsen beim Bandtransport drehen können.

Zur Vereinfachung der Darstellung sind Details zu dem Stechantrieb 6 und dem Antrieb der Wickeleinrichtung 5 in den Figuren 5 bis 8 nicht gezeigt. Diese Details können prinzipiell wie bei dem vorhergehenden Ausführungsbeispiel ausgebildet sein.

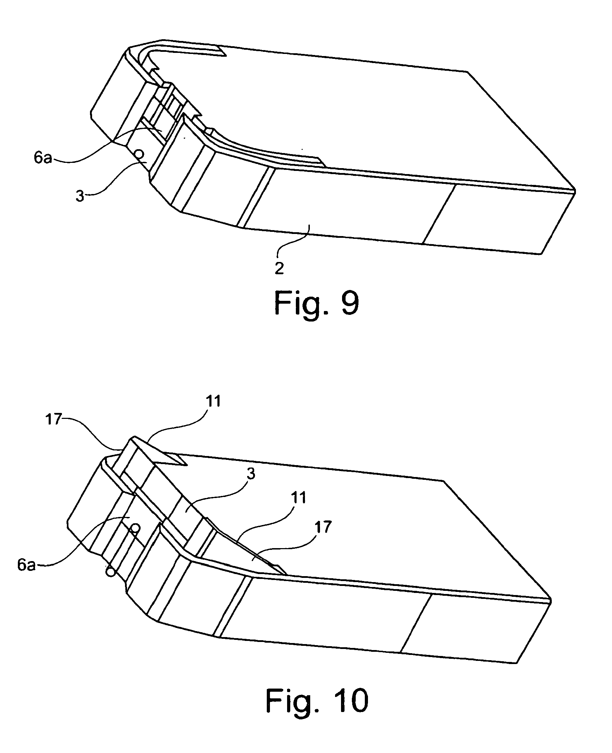

In den Figuren 9 und 10 ist ein weiteres Ausführungsbeispiel eines Stechsystems dargestellt, bei dem die Stichrichtung ähnlich wie bei dem Ausführungsbeispiel der Figuren 5 bis 8 parallel zu der geometrischen Achse erfolgt, um die sich die Wickeleinrichtung 5 zum Aufwickeln des Lanzettenträgerbandes 3 dreht. Die Transporteinrichtung weist zwei Schwenkarme 17 auf, zwischen denen sich die Stechposition befindet und die jeweils ein Bandführungselement 11 tragen, das in einer Biegung des Förderwegs angeordnet ist und um welches das Lanzettenträgerband 3 gebogen ist. Bei einer Stechbewegung führen die Schwenkarme 17 eine Schwenkbewegung aus und gelangen dabei aus der in Figur 9 gezeigten Stellung in die in Figur 10 gezeigte Stellung.

Das Bandführungselement 17 kann bei diesem Ausführungsbeispiel ebenso wie bei den vorstehend beschriebenen Ausführungsbeispielen ausgebildet sein. Möglich ist es beispielsweise auch, die Bandführungselemente als gekrümmte Führungsflächen auszubilden, auf den das Lanzettenträgerband 3 aufliegt und die sich in Förderrichtung erstrecken.

Die beiden Schwenkarme 17 können an ihren Enden verbunden sein und so einen hufeisenförmigen Bügel bilden. Bevorzugt berühren sich die Schwenkarme 17 jedoch nicht. Bei dem dargestellten Ausführungsbeispiel befinden sich zwischen den Schwenkarmen 17 die Stechposition und der Kopplungskopf 6a. Der Kopplungskopf 6a, der eine in der Stechposition angeordnete Lanzette 4 zusammen mit dem sie tragenden Bandabschnitt greift, führt jedoch ebenso wie bei den vorhergehend beschriebenen Ausführungsbeispielen eine lineare Bewegung aus.

Die Schwenkbewegung der Schwenkarme 17 erfolgt um eine geometrische Achse, die senkrecht zur Stichrichtung orientiert ist.

Das in den Figuren 9 und 10 gezeigte Ausführungsbeispiel ist eine Bandkassette, die das Lanzettenträgerband 3 und den Kopplungskopf 6a des Stechantriebs 6 enthält. Diese Bandkassette wird bestimmungsgemäß in ein passendes Aufnahmefach eines Stechgeräts eingesetzt, so dass der Stechantrieb 6 des Stechgeräts an den Kopplungskopf 6a der gezeigten Bandkassette ankoppelt. Das Stechgerät 6 enthält auch einen Antrieb zum Ankoppeln an die Wickeleinrichtung 5 einer eingesetzten Bandkassette, eine Biegeeinrichtung zum Biegen der Längskante des Bandes und ähnliches.

Die gezeigte Bandkassette bildet zusammen mit einem dazu passenden Stechgerät ein Stechsystem. Das Stechgerät kann dabei immer wieder verwendet werden, indem eine Bandkassette, bei der alle Lanzetten des Lanzettenträgerbandes benutzt wurden, gegen eine frische Bandkassette ausgetauscht wird.

In den Figuren 11 bis 14 ist schematisch ein Ausführungsbeispiel eines Stechsystems dargestellt, das nicht Teil der Erfindung ist bei dem die Bandführung ähnlich wie bei dem Ausführungsbeispiel der Figuren 1 bis 4 eine Umlenkung des Lanzettenträgerbandes 3 um 90° vorsieht. Die Wickeleinrichtung 5 des in Figuren 11 bis 14 dargestellten Ausführungsbeispiels ist mit einer Rücklaufsperre, beispielsweise einem Sperrklinkenmechanismus, gekoppelt, so dass sich die Wickeleinrichtung 5 nur vorwärts drehen kann. Die für eine Stechbewegung erforderliche Bandbeweglichkeit wird durch das elastische Verhalten des Lanzettenträgerbandes 3 bewirkt. Bei einer Stechbewegung dehnt sich das Lanzettenträgerband 3 elastisch und bewirkt so die erforderliche Verlängerung der Länge des Teils des Lanzettenträgerbandes 3 , der von der stechenden Lanzette 4 bis zu der Wickeleinrichtung 5 reicht.

In den Figuren 15 bis 18 ist ein nicht erfinderisches Ausführungsbeispiel eines Stechsystems dargestellt, bei dem die Bandführung der des in den Figuren 5 bis 8 dargestellten Ausführungsbeispiels entspricht. Bei einer Stechbewegung wird die Lanzette 4 parallel zu der geometrischen Drehachse, um die sich die Wickeleinrichtung 5 dreht, bewegt. Auch bei diesem Ausführungsbeispiel wird ein Rückwärtsdrehen der Wickeleinrichtung durch eine Rücklaufsperre verhindert und die erforderliche Beweglichkeit des Lanzettenträgerbandes 3 durch elastische Dehnung erreicht.

### Bezugszahlen

- 1: Stechsystem
- 2: Gehäuse
- 3: Lanzettenträgerband
- 4: Lanzette
- 5: Wickeleinrichtung
- 6: Stechantrieb
- 6a: Kopplungskopf des Stechantriebs
- 7: Körperteil
- 8: Vorratsrolle
- 9: Antriebselement
- 10a, 10b: Umlenkeinrichtung
- 11: Führungselement
- 12: Schrägfläche der Biegeeinrichtung
- 13: Zahnstange
- 14: Testfeld
- 15: Messeinrichtung
- 16: Gehäuseöffnung
- 17: Schwenkarm

## Patentansprüche

1. Stechsystem mit
- einem Lanzettenträgerband (3), das mehrere Lanzetten (4) trägt, die quer zu seiner Längsrichtung angeordnet sind,
- einer Transporteinrichtung (5, 11, 17), um das Lanzettenträgerband (3) in einer Förderrichtung entlang eines Förderwegs zu transportieren und dadurch Lanzetten (4) des Lanzettenträgerbandes (3) nacheinander in eine Stechposition zu bringen,
- einem Stechantrieb (6), um eine Lanzette (4), die sich in der Stechposition befindet, in eine Stechbewegung zu versetzen, und
- der Stechantrieb (6) bei einer Stechbewegung eine in die Stechposition gebrachte Lanzette (4) zusammen mit einem diese Lanzette (4) tragenden Abschnitt des Lanzettenträgerbandes (3) in Stichrichtung bewegt, und **dadurch gekennzeichnet, dass**
- mindestens ein in Förderrichtung hinter der Stechposition angeordnetes Teil der Transporteinrichtung (5, 11, 17), nachdem eine Lanzette (4) in die Stechposition gebracht wurde, vor oder während einer Stechbewegung dieser Lanzette (4) eine Bewegung ausführt zur Entspannung oder Freigabe des Lanzettenträgerbandes.

2. Stechsystem nach Anspruch 1, **dadurch gekennzeichnet, dass** die Transporteinrichtung (5, 11, 17) eine Wickeleinrichtung (5) umfasst, die durch schrittweises Aufwickeln des Lanzettenträgerbandes (3) Lanzetten (4) nacheinander in die Stechposition bringt.

3. Stechsystem nach Anspruch 2, **dadurch gekennzeichnet, dass** die Bewegung des mindestens einen Teils der Transporteinrichtung (5, 11) ein Rückwärtsdrehschritt der Wickeleinrichtung (5) ist, wobei die Wickeleinrichtung (5) einen Vorwärtsdrehschritt ausführt, um eine Lanzette (4) in die Stechposition zu bringen, und danach den Rückwärtsdrehschritt ausführt, um für eine Stechbewegung der in die Stechposition gebrachten Lanzette (4) Band von der Wickeleinrichtung (5) abzuwickeln.

4. Stechsystem nach Anspruch 3, **dadurch gekennzeichnet, dass** der Rückwärtsdrehschritt während der Stechbewegung erfolgt.

5. Stechsystem nach Anspruch 3 oder 4, **gekennzeichnet durch** ein Antriebselement (9), das bei dem Vorwärtsdrehschritt zum Antreiben der Wickeleinrichtung (5) mit der Wickeleinrichtung (5) koppelt und für den Rückwärtsdrehschritt von der Wickeleinrichtung (5) entkoppelt.

6. Stechsystem nach einem der vorstehenden Ansprüche, **dadurch gekennzeichnet, dass** die Transporteinrichtung (5, 11, 17) ein in Förderrichtung hinter der Stechposition angeordnetes Führungselement (11) umfasst, das in einer Biegung des Förderwegs angeordnet ist und um welches das Lanzettenträgerband (3) gebogen ist.

7. Stechsystem nach Anspruch 6, **dadurch gekennzeichnet, dass** das Bandführungselement (11) zwischen einer ersten Position, in der das Lanzettenträgerband (3) mit einem ersten Biegewinkel um das Bandführungselement (11) gebogen ist, und einer zweiten Position, in der das Lanzettenträgerband (3) weniger stark oder gar nicht gebogen ist, beweglich ist, und wobei das Bandführungselement (11) nach dem Positionieren einer Lanzette (4) in der Stechposition hin zur zweiten Position bewegt wird.

8. Stechsystem nach Anspruch 6 oder 7, **gekennzeichnet durch** zwei Bandführungselemente (11), zwischen denen sich die Stechposition befindet.

9. Stechsystem nach einem der vorstehenden Ansprüche, **dadurch gekennzeichnet, dass** die Transporteinrichtung (5, 11, 17) zwei Schwenkarme (17) aufweist, zwischen denen sich die Stechposition befindet und die jeweils ein Bandführungselement (11) aufweisen, das in einer Biegung des Förderwegs angeordnet ist und um welches das Lanzettenträgerband (3) gebogen ist, wobei die Schwenkarme bei einer Stechbewegung eine Schwenkbewegung ausführt.

10. Stechsystem nach Anspruch 9, **dadurch gekennzeichnet, dass** die Schwenkbewegung um eine geometrische Achse erfolgt, die senkrecht zur Stichrichtung orientiert ist.

11. Stechsystem nach einem der vorstehenden Ansprüche, **dadurch gekennzeichnet, dass** das Lanzettenträgerband (3) zwischen den Lanzetten (4) Testfelder (14) zur Untersuchung einer durch einen Lanzettenstich gewonnenen Körperflüssigkeitsprobe trägt.

12. Stechsystem nach einem der vorstehenden Ansprüche, **dadurch gekennzeichnet, dass** bei einer Stechbewegung die in Stichrichtung vordere Längskante des Lanzettenträgerbandes (3) umgebogen wird.

13. Stechsystem nach Anspruch 12, **dadurch gekennzeichnet, dass** sich die Spitze einer in die Stechposition gebrachten Lanzette (4) während der Stechbewegung von dem Lanzettenträgerband (3) abhebt.

14. Stechsystem nach einem der vorstehenden Ansprüche, **dadurch gekennzeichnet, dass** die Transporteinrichtung (5, 11, 17) über eine Rutschkupplung an ein Antriebselement (9) gekoppelt ist.

15. Stechsystem nach einem der vorstehenden Ansprüche, **dadurch gekennzeichnet, dass** der Stechantrieb (6) zur Kopplung an das Lanzettenträgerband (3) einen Kopplungskopf (6a) aufweist, der einen Spalt aufweist, durch den Lanzettenträgerband (3) hindurchgeführt ist.

## Claims

1. Lancing system having
a lancet carrier tape (3) that carriers a plurality of lancets (4) arranged transversely to its longitudinal direction;
a transport facility (5, 11, 17) for moving the lancet carrier tape (3) in a transport direction along a transport path and to thereby move lancets (4) of the lancet carrier tape (3) one after the other to a puncturing position; and
a lancing drive (6) for causing a lancet (4) that is in the puncturing position to perform a puncturing movement,
during a puncturing movement the lancing drive (6) moves a lancet (4), that has been brought to the puncturing position, in the puncturing direction together with a portion of the lancet carrier tape (3) that carries that lancet (4), **characterized in that**
once a lancet (4) has been moved to the puncturing position at least one element of the transport facility (5, 11, 17), arranged behind the puncturing position in the transport direction, performs a movement before or during the puncturing movement of that lancet (4) in order to ease tension or release the lancet carrier tape.

2. The lancing system as defined in Claim 1, **characterized in that** the transport facility (5, 11, 17) comprises a winding device (5) that successively brings lancets (4) to the puncturing position by winding up the lancet carrier tape (3) by steps.

3. The lancing system as defined in Claim 2, **characterized in that** the movement of the at least one part of the transport facility (5, 11) is a reverse rotary step of the winding device (5), and that the winding device (5) performs a forward rotary step to move a lancet (4) to the puncturing position, and then performs the reverse rotary step to unwind a length of tape from the winding device (5) for a puncturing movement of the lancet (4) that has been moved to the puncturing position.

4. The lancing system as defined in Claim 3, **characterized in that** the reverse rotary step is effected during the puncturing movement.

5. The lancing system as defined in Claim 3 or Claim 4, **characterized by** a driving element (9), which engages the winding device (5) during the forward rotary step to drive the winding device (5), and is uncoupled from the winding device (5) for the reverse rotary step.

6. The lancing system as defined in any of the preceding claims, **characterized in that** the transport means (5, 11, 17) comprises at least one guide element (11), arranged behind the puncturing position in the transport direction, which is located in a bend of the transport path and about which the lancet carrier tape (3) is bent.

7. The lancing system as defined in Claim 6, **characterized in that** the tape-guiding element (11) can be moved between a first position in which the lancet carrier tape (3) is bent about the tape-guiding element (11) at a first bending angle, and a second position in which the lancet carrier tape (3) is bent to a lesser degree or not at all, the tape-guiding element (11) being moved toward the second position once a lance (4) has been located in the puncturing position.

8. The lancing system as defined in Claim 6 or Claim 7, **characterized by** two tape-guiding elements (11) between which is the puncturing position.

9. The lancing system as defined in any of the preceding claims, **characterized in that** the transport facility (5, 11, 17) comprises two pivot arms (17) that enclose between them the puncturing position and each of which comprises a tape-guiding element (11) which is arranged in a bend of the transport path and about which the lancet carrier tape (3) is bent, the pivot arms performing a pivoting movement during a puncturing movement.

10. The lancing system as defined in Claim 9, **characterized in that** the pivoting movement is effected about a geometric axis oriented at a right angle to the puncturing direction.

11. The lancing system as defined in any of the preceding claims, **characterized in that** test fields (14) are provided on the lancet carrier tape (3) between the lancets (4) for analyzing a sample of a body liquid collected by the puncturing operation.

12. The lancing system as defined in any of the preceding claims, **characterized in that** the forward longitudinal edge of the lancet carrier tape (3), viewed in the puncturing direction, is bent over during a puncturing movement.

13. The lancing system as defined in Claim 12, **characterized in that** the tip of the lancet (4) that has been moved to the puncturing position is lifted off the lancet carrier tape (3) during the puncturing movement.

14. The lancing system as defined in any of the preceding claims, **characterized in that** the transport facility (5, 1, 17) is coupled with a drive element (9) via a slip clutch.

15. The lancing system as defined in any of the preceding claims, **characterized in that** for coupling the lancing drive (6) with the lancet carrier tape (3), the lancing drive is provided with a coupling head (6a) comprising a gap through which the lancet carrier tape (3) is guided.

## Revendications

1. Système piqueur comprenant
- une bande support à lancettes (3), qui porte plusieurs lancettes (4), qui sont disposées transversalement à sa direction longitudinale,
- un système de transport (5, 11, 17), pour transporter la bande support à lancettes (3) dans un sens de transport le long d'une course de transport et amener ainsi des lancettes (4) de la bande support à lancettes (3) de façon successive dans une position de piqûre,
- une commande de piqûre (6), afin de mettre une lancette (4) se trouvant dans la position de piqûre dans un mouvement de piqûre, et
- la commande de piqûre (6) déplaçant lors d'un mouvement de piqûre une lancette (4) amenée dans la position de piqûre en même temps qu'une partie, portant cette lancette (4), de la bande support à lancettes (3) dans le sens de piqûre, et **caractérisé en ce que**
- au moins une partie, disposée dans le sens de transport en aval de la position de piqûre, du dispositif de transport (5, 11, 17) exécute un mouvement avant ou pendant un mouvement de piqûre de cette lancette (4), une fois qu'une lancette (4) a été amenée dans la position de piqûre, pour le soulagement ou la libération de la bande support à lancettes.

2. Système piqueur selon la revendication 1, **caractérisé en ce que** le dispositif de transport (5, 11, 17) comprend un dispositif d'enroulement (5), qui amène des lancettes (4) de façon successive dans la position de piqûre par enroulement progressif de la bande support à lancettes (3).

3. Système piqueur selon la revendication 2, **caractérisé en ce que** le déplacement de la au moins une partie du dispositif de transport (5, 11) est une étape de rotation arrière du dispositif d'enroulement (5), le dispositif d'enroulement (5) exécutant une étape de rotation avant, afin d'amener une lancette (4) dans la position de piqûre, et exécute ensuite l'étape de rotation arrière, afin de dérouler de la bande du dispositif d'enroulement (5) pour un mouvement de piqûre de la lancette (4) amenée dans la position de piqûre.

4. Système piqueur selon la revendication 3, **caractérisé en ce que** l'étape de rotation arrière s'effectue pendant le mouvement de piqûre.

5. Système piqueur selon la revendication 3 ou 4, **caractérisé par** un élément d'entraînement (9), qui est couplé avec le dispositif d'enroulement (5) lors de l'étape de rotation en avant pour l'entraînement du dispositif d'enroulement (5) et est dissocié du dispositif d'enroulement (5) pour l'étape de rotation arrière.

6. Système piqueur selon l'une des revendications précédentes, **caractérisé en ce que** le dispositif de transport (5, 11, 17) comprend un élément de guidage (11) disposé dans le sens de transport en aval de la position de piqûre, lequel élément est disposé dans une courbure de la course de transport et autour duquel la bande support à lancettes (3) est pliée.

7. Système piqueur selon la revendication 6, **caractérisé en ce que** l'élément de guidage de bande (11) peut être déplacé entre une première position, dans laquelle la bande support à lancettes (3) est pliée avec un premier angle de flexion autour de l'élément de guidage de bande (11), et une seconde position, dans laquelle la bande support à lancettes (3) est moins fortement pliée ou pas du tout pliée, et l'élément de guidage de bande (11) étant déplacé vers la seconde position après le positionnement d'une lancette (4) en direction de la position de piqûre.

8. Système piqueur selon la revendication 6 ou 7, **caractérisé par** deux éléments de guidage de bande (11), entre lesquels se trouve la position de piqûre.

9. Système piqueur selon l'une des revendications précédentes, **caractérisé en ce que** le dispositif de transport (5, 11, 17) présente deux bras basculants (17), entre lesquels se trouve la position de piqûre et qui présentent chacun un élément de guidage de bande (11), qui est disposé dans une courbure de la course de transport et autour duquel la bande support à lancettes (3) est pliée, les bras basculants exécutant un mouvement de basculement lors d'un mouvement de piqûre.

10. Système piqueur selon la revendication 9, **caractérisé en ce que** le mouvement de basculement s'effectue autour d'un axe géométrique, qui est orienté perpendiculairement à la direction de piqûre.

11. Système piqueur selon l'une des revendications précédentes, **caractérisé en ce que** la bande support à lancettes (3) porte des zones test (14) entre les lancettes (4) pour l'examen d'un échantillon de liquide corporel obtenu par une piqûre de lancette.

12. Système piqueur selon l'une des revendications précédentes, **caractérisé en ce que**, lors d'un mouvement de piqûre, l'arête longitudinale avant dans le sens de piqûre, de la bande support à lancettes (3) est repliée.

13. Système piqueur selon la revendication 12, **caractérisé en ce que** la pointe d'une lancette (4) amenée dans la position de piqûre se soulève de la bande support à lancettes (3) pendant le mouvement de piqûre.

14. Système piqueur selon l'une des revendications précédentes, **caractérisé en ce que** le dispositif de transport (5, 11, 17) est couplé par un accouplement à glissement à un élément d'entraînement (9).

15. Système piqueur selon l'une des revendications précédentes, **caractérisé en ce que** la commande de piqûre (6) présente, pour le couplage avec la bande support à lancettes (3), une tête d'accouplement (6a) présentant une fente, à travers laquelle la bande support à lancettes (3) est guidée.
